(19) Europäisches Patentamt
European Patent Office
Office européen des brevets

(11) EP 2 028 473 B1

## (12) EUROPÄISCHE PATENTSCHRIFT

(45) Veröffentlichungstag und Bekanntmachung des Hinweises auf die Patenterteilung:
**02.05.2012 Patentblatt 2012/18**

(51) Int Cl.:
**G01N 3/40** *(2006.01)*          **G01N 33/46** *(2006.01)*

(21) Anmeldenummer: **07450141.2**

(22) Anmeldetag: **20.08.2007**

(54) **Verfahren zur Untersuchung und Beurteilung der Standfestigkeit von Holzmasten**

Method for inspecting and assessing the stability of wooden masts

Procédé destiné à la recherche et à l'évaluation de la solidité de mâts en bois

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**25.02.2009 Patentblatt 2009/09**

(73) Patentinhaber: **BEA Electrics Energietechnik GmbH**
**1230 Wien (AT)**

(72) Erfinder: **Sched, Wolfgang**
**1230 Wien (AT)**

(74) Vertreter: **Margotti, Herwig Franz et al**
**Schwarz & Partner**
**Patentanwälte**
**Wipplingerstrasse 30**
**1010 Wien (AT)**

(56) Entgegenhaltungen:
WO-A-98/47671          DE-A1- 4 122 494
DE-A1- 19 716 817     DE-C1- 19 516 643
FR-A- 2 760 842          GB-A- 2 256 054
JP-A- 58 018 165          US-A- 6 158 272

**Beschreibung**

Die Erfindung geht aus von einem Verfahren zur Untersuchung und Beurteilung der Standfestigkeit von Holzmasten, insbesondere von Telefon- und Strommasten.

Holzmasten sind im Bereich des Mittel- und Niederspannungsleitungsbaus sowie des Telefonwesens nach wie vor nicht wegzudenken. Ein Ersatz der Holzmasten durch Beton- oder Metallmasten ist in Bezug auf die Haltbarkeit und die benötigte Elastizität der Leitungsaufhängung bzw. in Bezug auf die Wirtschaftlichkeit unbefriedigend. Holzmasten sind bei entsprechender Vorbehandlung/Imprägnierung und regelmäßiger Wartung über lange Zeiträume zuverlässige Träger für die Leitungen. Trotz der Imprägnierung kommt es jedoch nach einiger Zeit trotzdem zu Beschädigungen, sei es schleichend durch feuchtigkeitsbedingte Fäulnis, den Befall mit Pilzen oder Insekten oder durch plötzliche Ereignisse wie Blitzschläge.

Aus der Praxis sind Verfahren bekannt, bei welchen entweder eine oberflächliche visuelle Kontrolle oder eine Probebohrung im Außenbereich des Masten mit einer Beurteilung des erbohrten Bohrkerns durchgeführt wird. Die erste Methode hat dabei den Nachteil, über Beschädigungen im Inneren des Masten, beispielsweise durch Befall von Pilzen und Insekten, aufsteigende Feuchtigkeit etc. keinerlei Aussage zu liefern, während die Beurteilung von Bohrkernen eine große Streuung in der Genauigkeit aufweist, da Schadstellen durch diese punktuellen einzelnen Bohrungen oft nicht erreicht werden. In der Folge sind Schäden durch umstürzende Masten sowohl am Leitungsnetz als auch in der Umgebung der Masten zu befürchten.

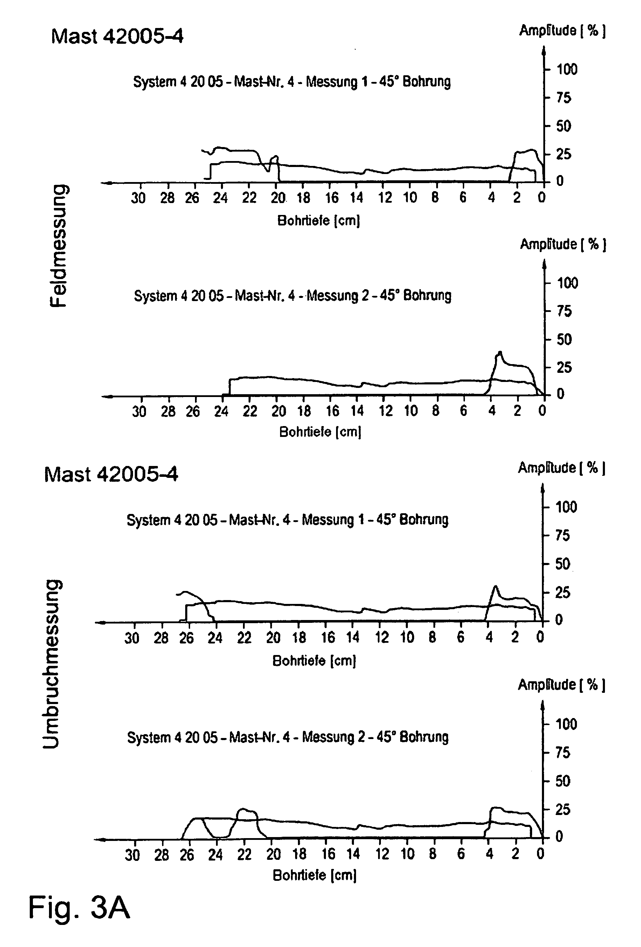

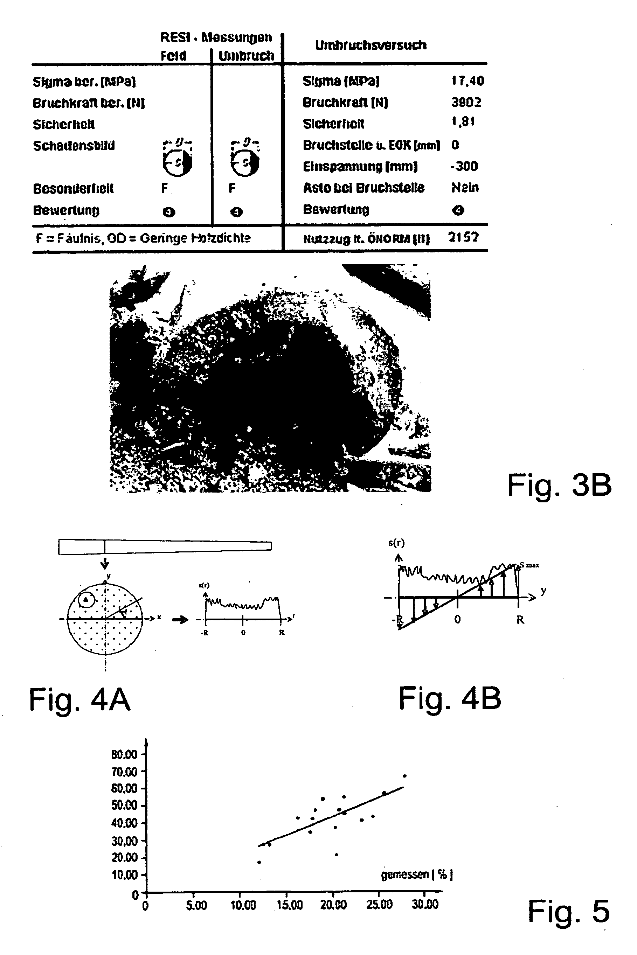

WO01/65253 A2 beschreibt ein Verfahren zur Untersuchung der Stabilität von Holzmasten durch Messung des Bohrwiderstandes bzw. der Holzdichte in Abhängigkeit von der Eindringtiefe, wobei durch Vergleich mit statistisch ermittelten Referenzkennlinien für Holzproben mit unterschiedlichen Schadensbildern eine Belastbarkeitsgrenze ermittelt sowie eine Bewertung der inneren Struktur des Holzes vorgenommen wird. Dabei werden in der Ausführungsform der Abbildungen 3 und 4 an dem Holzmasten Bohrungen in der gleichen horizontalen Schnittebene, jedoch unter unterschiedlichen Winkeln vorgenommen.

FR-A-2 760 842_offenbalt ein Verfahren zur Untersuchung und Beurteilung der Standfestigkeit von Holzmasten, wobei Messkonnlinien duch Bohrwiderstandsmessungen am Holzmast ermittelt werden. Die zusätzliche Ermittlung von Referenzkennlinien des Bohrwilerstandes ermöglicht eine Ermittlung von Schadstellen in den Holzmasten.

DE 41 22 494 A1 offenbart ein ähnliches Verfahren, das allerdings für die Holzprüfung bei Fachwerkgebäuden optimiert ist.

Es ist die Aufgabe der Erfindung, ein Verfahren zur Untersuchung und Beurteilung der Standfestigkeit von Holzmasten anzugeben, welches auf einfache Weise aussagekräftige Werte zur Beurteilung des inneren Zustandes und somit der Standfestigkeit der Holzmasten liefert.

Die Aufgabe wird durch das erfindungsgemäße Verfahren gemäß Anspruch 1 gelöst, welches unter anderem die folgenden Merkmale aufweist:

- Ermittlung von Messkennlinien durch Bohrwiderstandsmessungen, die dem tatsächlichen Bohrwiderstand entsprechen, bzw. einer daraus abgeleiteten Größe für die Holzfestigkeit bzw. Holzdichte,
- Ermittlung von Referenzkennlinien des Bohrwiderstandes bzw. einer daraus abgeleiteten Größe der unteren Grenze für die Belastbarkeit, und
- Ermittlung von Schadstellen, Schadensbildern und Schadensklassen aus dem Vergleich der Messkennlinien der Bohrwiderstandsmessungen und der Referenzkennlinien.

Vorteilhafterweise ist es dadurch möglich, mittels Kombination einfacher Messvorgänge mit statistisch ermittelten Referenzen eine sichere Beurteilung des Zustandes eines Holzmasten zu erhalten und damit schadhafte Holzmasten gezielt sanieren oder austauschen zu können.

Vorteilhafte Weiterbildungen und Ausgestaltungen des erfindungsgemäßen Verfahrens gehen aus den Unteransprüchen hervor.

Eine weitere Ausführungsform des Verfahrens umfasst weiterhin die Merkmale einer Sichtkontrolle der Holzmasten, der Kombination der Ergebnisse der Bohrwiderstandsmessungen mit den Ergebnissen der Sichtkontrollen, und der Festlegung der Intervalle für weitere Maßnahmen, so dass vorteilhafterweise eine sehr umfassende Befundung der Holzmasten möglich ist.

Für verschiedene Holzarten werden jeweils eigene Referenzkennlinien aufgenommen, so dass den Unterschieden in der Holzstruktur Rechnung getragen werden kann.

Um eine statistisch aussagekräftige Referenzkennlinie zu erhalten, werden vorteilhafterweise für jede Holzart mindestens fünfzig Messkennlinien aufgenommen und daraus eine Referenzkennlinie ermittelt.

Zur Bestimmung eines unteren noch zulässigen Grenzwerts der Bruchfestigkeit eines Holzmasten wird eine 5%-Fraktile der Gaußschen Normalverteilung für die Referenzkennlinien herangezogen, welche eine einfache und trotzdem zufriedenstellende Abschätzung ermöglicht.

Vorteilhafterweise erfolgt der Vergleich einer Referenzkennlinie mit einer Messkennlinie abschnittsweise.

Eine Berechnung und ein Vergleich der Flächen unter den Kennlinien erfolgt dabei in vorgegebenen Schritten, vorzugsweise in 2,5%-Schritten, was eine ausreichend hohe Genauigkeit bei kurzer Auswertungszeit zur Folge hat.

Aus dem Vergleich einer Referenzkennlinie mit einer Messkennlinie kann eine Zuordnung zu einem Schadensbild und in eine Schadensklasse erfolgen, worauf zuverlässig bestimmt werden kann, ob ein Holzmast gepflegt oder ersetzt werden muss.

Um eine aussagekräftige Messung zu erhalten, werden die Bohrwiderstandsmessungen an den Holzmasten in zumindest zwei im Winkel zueinander eingebrachten Bohrungen durchgeführt.

Vorzugsweise werden an einem eingegrabenen Holzmast zwei 45°-Bohrungen im Erdübergangsbereich durchgeführt, davon eine Bohrung in einer Leitungsrichtung und eine Bohrung 90° zur Leitungsrichtung, um den kritischen Bereich, in welchem der Holzmast am anfälligsten für Fäulnis ist, zu begutachten.

An einem Holzmast mit Betonfuß werden bevorzugt zwei waagerechte Messungen zwischen den Befestigungsschrauben des Holzmasts durchgeführt, davon eine Bohrung in einer Leitungsrichtung und eine Bohrung 90° zur Leitungsrichtung.

Vorzugsweise wird die Bohrwiderstandsmessung mittels eines Bohrers mit angeschliffener Spitze und einem Durchmesser von 1 bis 3 mm ausgeführt, welcher eine nicht relevante Beschädigung bzw. Schwächung des Holzes mit sich bringt.

Im Folgenden wird die Erfindung anhand von nicht einschränkenden Ausführungsbeispielen unter Bezugnahme auf die Zeichnungen näher erläutert. In den Figuren zeigen:

Fig. 1          schematische Schnittdarstellungen durch Holzmasten mit verschiedenen, typischen Schadensbildern,

Fig. 2A-B     beispielhaft eine Referenzkennlinie eines Holzmasten aus Kiefernholz sowie die Referenzkennlinie mit einer darübergelegten Messkennlinie aus einer Feldmessung an einem solchen Holzmasten,

Fig. 3          einen beispielhaften vollständigen Mess- und Beurteilungsvorgang für einen Holzmasten,

Fig. 4A-B     die Physik der zugrundeliegenden Umbruchversuche beschreibende Diagramme,

Fig. 5          ein Diagramm zur Ermittlung eines Skalierungsfaktors für die Skalierung der Messwerte aus den Bohrwiderstandsmessungen, und

Fig. 6A-C     eine vergrößerte Darstellung zweier Bohrwiderstandsmesskurven sowie des dazugehörigen Mastquerschnitts einer beispielhaften Mastbefundung.

Die herkömmlichen Wartungsarbeiten der Holzmasten wurden in der Vergangenheit entweder auf reine Sichtkontrollen des Mastzustandes oder auf Bohrungen mit einer anschließenden Sichtkontrolle des Bohrkerns beschränkt. Die durch die letztgenannte Methode erhobenen Ergebnisse sind jedoch unvollständig und daher sehr unsicher, da stets nur stichprobenartig gebohrt und meist nur eine Bohrung pro Mast gesetzt wurde. Dementsprechend kann beispielsweise eine nur teilweise ausgeprägte Fäulnis im Mastinneren nicht erkannt werden, wenn die Bohrung nur gesundes Holz getroffen hat.

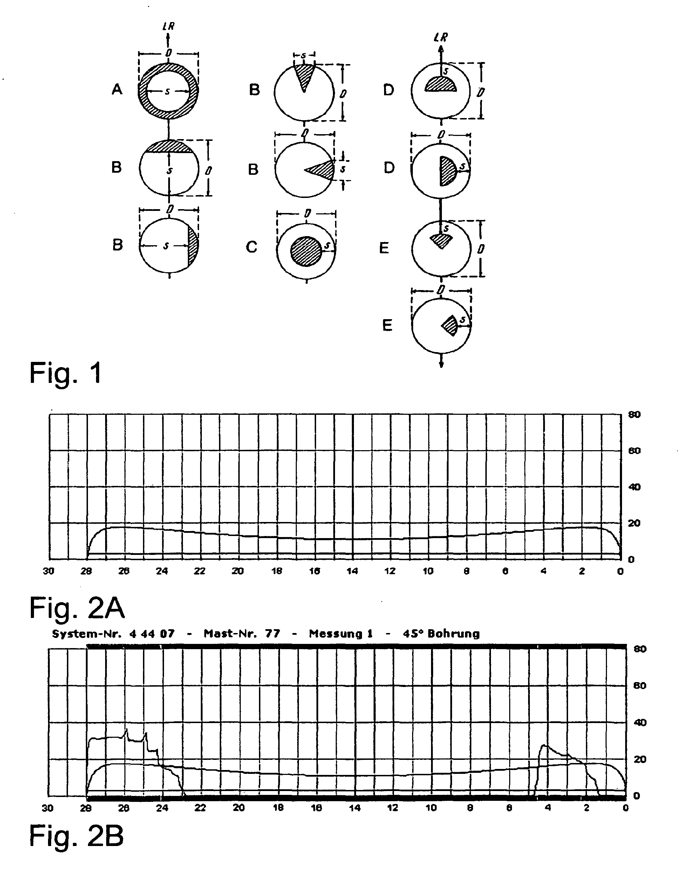

Aus den Bohrmessungen sowie aus Umbruchversuchen an schadhaften Holzmasten sind Schadensklassen definiert worden, die seit langem Anwendung finden und auch dem erfindungsgemäßen Verfahren zur Untersuchung und Beurteilung der Standfestigkeit von Holzmasten zugrunde liegen. In Fig. 1 sind typische Schadensbilder bei Holzmasten dargestellt, welche die Grundlage für die Einstufung in die Schadensklassen sind, worauf weiter unten noch detaillierter eingegangen wird. Die Definition der Schadensklassen erfolgt dabei über eine äußere Inaugenscheinnahme sowie die Messung des Biegemoments sowie von Nutzzug und Belastung an der Mastspitze.

Weitere Parameter zur Einteilung in Schadensklassen sind messbare Parameter wie der Durchmesser und die Masthöhe, das Schwächungsmaß, die Spannweite der Leitungen zwischen den Masten und die Anzahl und Querschnitte der Leitungen, sowie tabellarische Parameter wie Grenzmaße, Schwächungsfaktoren, zulässige Biegemomente, Windkräfte und sonstige Belastungen.

Die eigentliche Messung gemäß dem erfindungsgemäßen Verfahren am Holzmasten erfolgt in mehreren Schritten. Zunächst werden die korrekten Bohrmesspunkte an dem zu untersuchenden Holzmast aufgesucht. Diese liegen bei einem im Untergrund eingegraben verankerten Holzmast im Erdübergangsbereich, da dieser Bereich besonders anfällig für Fäulnis und Schädlingsbefall ist. Dort werden zumindest zwei 45°-Bohrungen bezogen auf eine Waagerechte gesetzt, deren eine in einer Leitungsrichtung, in welcher sich die an dem Holzmasten aufgehängten Leitungen erstrecken, und deren zweite unter einem Winkel von 90° zur Leitungsrichtung in den Holzmast eingebracht wird. Für einen mittels eines Betonfußes verankerten Holzmasten werden zumindest zwei 90°-Bohrungen zwischen den Befestigungen des Holzmasten an dem Betonfuß in den Holzmast gesetzt, davon wiederum eine Messung in Leitungsrichtung und eine Messung 90° zur Leitungsrichtung.

Bei der Bohrwiderstandsmessung wird eine Bohrnadel mit einem Durchmesser von ca. 1 bis 3 mm mit angeschliffener Spitze in das Holz eingebohrt. Bei zunehmender Härte des Holzes und damit höherem Bohrwiderstand ist eine größere Leistung des Vorschubmotors erforderlich, die im Gerät gemessen und als Kurve über der Eindringtiefe aufgezeichnet wird (Kraft-Weg-Diagramm). Die Messgeräte sind mit einer Elektronikeinheit und entsprechender Auswertungssoftware ausgestattet. Die Messdaten lassen sich so auf einen PC übertragen und weiterverarbeiten. Das Holz wird bei dieser Messung nur geringfügig verletzt. Die Bohrfasern bleiben im Bohrloch und verschließen den Bohrkanal wieder.

Durch die Härteunterschiede des Früh- und Spätholzes innerhalb eines Jahresringes entsteht eine holztypische Bohrkurve. Wenn das zu untersuchende Holz eine Zerstörung aufweist, d.h. die Materialstruktur geschwächt ist, wird dies an einem entsprechenden Abfall der Bohrwiderstandskurve deutlich. Da die Härte des Holzes mit seiner Dichte und diese wiederum mit den wichtigsten Festigkeits- und Zustandsparametern korreliert, ergibt sich aus dem Verlauf der Bohrwiderstandskurve eine aussagekräftige Darstellung über den Zustand des Holzes an der durchbohrten Stelle.

Die Messwerte des Gerätes werden auf elektronischem Weg gespeichert, nach Abschluss der Messungen auf einen PC übertragen und dort weiterbearbeitet. Auf diesem Weg ist es somit möglich, eine Auswertung durchzuführen, welche dann eine Interpretation und entsprechende Schlussfolgerungen in Bezug auf die Sanierung oder den Ersatz der Holzmasten erlaubt.

Die Aufbereitung und Auswertung der Messdaten sowie die Einteilung in verschiedene Schadensklassen kann auf elektronischem Weg unter Nutzung herkömmlicher Datenverarbeitungsanlagen sowie geeigneter Programme erfolgen. Jeder Holzmast kann in einer entsprechenden Datenbank anhand des Ergebnisses der Messkurven und weiterer objektspezifischer Parameter wie Mastdurchrnesser, Höhe, Feldlänge etc. sowie Parametern aus dem Begehungsprotokoll wie Spechtlöcher, Blitzschlag etc. aufgefunden und hinsichtlich seiner Standfestigkeit bewertet werden. Bemerkungen über fällige Sanierungsintervalle oder einen Austausch ergänzen den jeweiligen Datensatz.

Die Bohrwiderstandsmessung ist bereits per se eine aussagekräftige Meßmethode. Bei stark schadhaften Holzmasten, bei welchen bereits die erste Bohrwiderstandsmessung einen hohen Zerstörungsgrad enthüllt, kann eine weitergehende Untersuchung u.U. entfallen. Erfindungsgemäß ist jedoch zur Quantifizierung der Messergebnisse vorgesehen, eine Ermittlung von Referenzkennlinien an gesundem Holz durchzuführen und die Kennlinien der Bohrwiderstandsmessungen mit den Referenzkennlinien zu vergleichen, um nicht nur eine qualitative, sondern auch eine quantitative Einschätzung des Zustandes von Holzmasten zu ermöglichen.

Die Werkstofffestigkeit ist proportional zur Holzdichte und damit proportional zum Bohrwiderstand. Somit müssen unterschiedliche Referenzkennlinien für verschiedene Holzarten aufgenommen werden, um den Unterschieden in der Holzstruktur der jeweiligen Holzart Rechnung tragen zu können. Um eine statistisch signifikante Aussage treffen zu können, werden zur Ermittlung einer Referenzkennlinie Aufnahmen von mindestens 50 Messkennlinien für jeden Typ durchgeführt. Durch Ermittlung der 5%-Fraktile der Gaußschen Normalverteilung wird die Referenzkennlinie für den unteren noch zulässigen Grenzwert bestimmt.

Fraktile bezeichnen dabei Werte, die mit angegebener Wahrscheinlichkeit über- oder unterschritten werden. So kann die 50%-Fraktile mit gleicher Wahrscheinlichkeit über-, aber auch unterschritten werden. Bei symmetrischer Verteilung entspricht die 50%-Fraktile dem Mittelwert der Eigenschaften. Die 5%-Fraktile wird statistisch von 5% der Werte unterschritten, die 95%-Fraktile von 5% überschritten.

Ein Vergleich zwischen einer solchermaßen ermittelten Referenzkennlinie und einer beispielhaften Bohrmessungskennlinie ist in Fig. 2A und 2B dargestellt. Fig. 2A zeigt eine Referenzkennlinie für einen Holzmast aus Kiefernholz. Für andere Holzarten ist die Referenzkennlinie anders gestaltet, für härteres Holz beispielsweise ist die auf der Ordinate der Diagramme angegebene Amplitude höher, da ein höherer Bohrwiderstand vorliegt.

Der Vergleich der Referenzkennlinie mit einer Messkennlinie erfolgt zunächst durch einen einfachen Vergleich, indem die Messkennlinie über die Referenzkennlinie gelegt und zunächst visuell verglichen wird. Des weiteren kann jedoch auch über eine Integration eine Berechnung und ein Vergleich der Flächen unter den Kennlinien beispielsweise in 2,5%-Schritten erfolgen. Somit ist eine sehr aufschlussreiche und detaillierte Ermittlung der Faulstellen, Schadensbilder und Schadensklassen aus den Messkennlinien möglich.

Abschließend kann auch eine Kombination der Ergebnisse der Bohrwiderstandsmessungen mit den Ergebnissen der Sichtkontrollen erfolgen, um das Bild abzurunden.

Ein beispielhafte Vollbegutachtung eines Holzmasten ist in Fig. 3 schematisch dargestellt. Links sind die Bohrwiderstandskurven von insgesamt vier Bohrungen an dem exemplarischen Holzmast dargestellt. Aus den Bohrwiderstandskurven geht hervor, dass eine umfangreiche Zone mit Fäulnis in dem Holzmast vorliegt.

Aus den Messergebnissen lässt sich ein Schadensbild eruieren, welches schematisch oben in Fig. 3B dargestellt ist. Der Vergleich mit den bekannten Schadensklassen aus Fig. 1 zeigt eine hohe Übereinstimmung mit einem bekannten Schadensbild. Die Einstufung der beispielhaften Befundung erfolgt in Schadensklasse IV, d.h. der befundete Holzmast ist zu ersetzen. Unten ist ein Querschnitt durch den aufgrund der eruierten Schadensklasse ersetzten Holzmast gezeigt.

Um eine vollständige Mastbefundung zu ermöglichen, muss jedoch eine Skalierung der Bohrwiderstände erfolgen, was durch die Errechnung der Bruchspannung σ erfolgt. Wie diese ermittelt wird, wird im Folgenden ausführlich beschrieben.

Zunächst erfolgt die Berechnung der maximalen Biegespannung für unskalierte Bohrwiderstände, dann erfolgt ein Vergleich mit aus Umbruchversuchen ermittelten empirischen Werten, woraus sich ein Skalierungsfaktor für die Bohrwiderstände ermitteln lässt.

Das zulässige Biegemoment für eine Fläche A, in diesem Fall eine Querschnittsfläche eines zu befundenden Masten, ist bekanntermaßen definiert durch:

$$M_x = \int_A \sigma_y(r).y.dA = \int_A \sigma_{max}.\frac{y^2}{R}.dA.$$

In polaren Koordinaten ausgedrückt ist dA=r.dr.dφ und y=r.sin φ.

Daraus folgt, dass $M_x = \int_0^\pi \int_{-R}^R (\sigma(r).\frac{(r.\sin\varphi)^2}{R}).r.dr.d\varphi$ bzw. $M_x = \int_{-R}^R \frac{\pi.\sigma(r).r^3}{2.R}.dr$ ist, woraus $M_x = \frac{\pi.\Delta_r}{2.R}.\sum_{i=1}^n \sigma_i.r_i^3$ mit dem Radius r als absolutem Wert folgt.

Die maximale Biegespannung berechnet sich dann aus $\sigma = \frac{M}{W} = \frac{M}{\frac{\pi.R^3}{4}} = \frac{2.\Delta_r}{R^4}.\sum_{i=1}^n \sigma_i.r_i^3$ wiederum mit dem Radius r als absolutem Wert.

Die Fig. 4A und 4B zeigen die entsprechenden grafischen Darstellungen der physikalischen Zusammenhänge beim Umbrechen eines Masten im untersten Viertel der Mastlänge. Eine Auswertung der aus möglichst vielen Umbruchversuchen gewonnenen Daten lässt eine Trendlinie erkennen, welche durch

$$y = 2{,}1842 * x \quad \Rightarrow \quad y = 2{,}2*x$$

beschrieben werden kann. Der Wert von 2,1842 entspricht dabei der Steigung einer durch die Vielzahl von Messpunkten gelegten Geraden, wie in Fig. 5 dargestellt. Je mehr Umbruchsversuche durchgeführt werden, desto präziser kann der Skalierungsfaktor bestimmt werden.

Daraus kann die Skalierung der Bohrwiderstände mit dem aus den Umbruchsversuchen empirisch ermittelten Skalierungswert vorgenommen werden:

$$\sigma\,[MPa] = 2{,}2 * \sigma\,[\%],$$

wobei der Wert σ aus den Bohrwiderstandsmessungen ermittelt wird.

Somit ergibt sich aus obigen Formeln:

$$\sigma\,[MPa] = 2{,}2 * \frac{2.\Delta_r}{R^4}.\sum_{i=1}^n \sigma_i[\%].r_i^3 \quad \text{mit dem Radius r als absolutem Wert.}$$

In der weiteren Bewertung des Masten müssen diverse Kriterien betrachtet werden. Die Amplitude bei der Bohrwiderstandsmessung gibt Aufschluss über die Holzqualität. Ein fäulnisbedingter Hohlraum liegt beispielsweise vor, wenn die gemessene Amplitude kleiner als 2,5% der Referenzkennlinie wird. Bei geringer Holzdichte sinkt die Amplitude ebenfalls unter die Referenzkennlinie, dies bedingt durch die intakte Holzstruktur ohne Fäulnis jedoch kein Schadensbild. Ein Schadensbild liegt vor, wenn bei der Messkurve mind. 2,5 mm fäulnisbedingter Hohlraum ablesbar sind.

Die Auswertung der Messwerte der Bohrwiderstandsmessungen erfolgt dann in mehreren Schritten. Zunächst muss der durchschnittliche Sigma-Wert aus den beiden Bohrwiderstandsmessungen gemäß einer einfachen Mittelung erfolgen:

$$\sigma_{mittel} = \frac{\sigma_{Messung\ 1} + \sigma_{Messung\ 2}}{2}$$

Die eigentliche Bewertung erfolgt dann anhand des berechneten $\sigma$-Wertes, Eine einfache Sicherheit gemäß EN-Norm schreibt einen Wert von $\sigma = 15$ MPa (N/mm$^2$) vor. Entsprechend ergeben sich Standsicherheitsklassen ohne Schadensbilder:

| Klasse | Bruchlast [N/mm$^2$] | Standsicherheit |
|--------|----------------------|-----------------|
| 1 | $\geq 45$ | Mindestens 3-fach |
| 2 | 37,5 - 45 | 2,5 - 3-fach |
| 3 | 30 - 37,5 | 2 - 2,5-fach |
| 4 | < 30 | Weniger als 2-fach |

Ist ein Schadensbild aus der Messkurve erkennbar, erfolgt zunächst eine Bewertung anhand eines Schadensbildes gemäß der bekannten "VDE 0210 - Vorschriften über Freileitungen", indem das entsprechende Schadensbild ermittelt wird, wie in Fig. 1 bereits kurz beschrieben. Die Schadensbilder aus Fig. 1 sind dabei in äußere Schadensbilder (mit A und B bezeichnete Schadensbilder) sowie innere Schadensbilder (mit C, D und E bezeichnete Schadensbilder) unterteilt, je nachdem, ob der Schaden äußerlich sichtbar ist oder nicht.

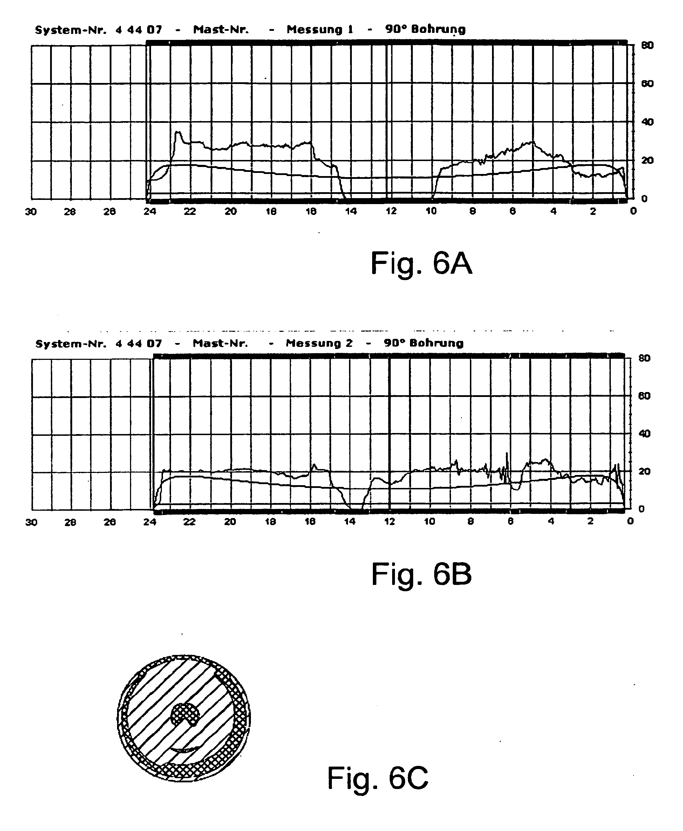

Das Schadensbild entsteht durch Interpolation der beiden y-Achsenwerte der beiden Messungen am jeweiligen x-Achsenwert. Es werden zuerst die vier Quadranten der Messung gesondert betrachtet (Fig. 6A bis 6C). Als Quadrant wird in diesem Zusammenhang die halbe Länge jeder der beiden, im 90°-Winkel zueinander versetzt in den Mastfuß eingebrachten Bohrwiderstandsmessungen bezeichnet, so dass die beiden sich kreuzenden Bohrwege in je zwei Quadranten (1. und 3. sowie 2. und 4. Quadrant) zerfallen.

Wenn eine Faulstelle mit mindestens 2,5 mm Hohlraum vorhanden ist und diese überwiegend in den äußeren 40% des jeweiligen Quadranten liegt, dann liegt für diesen Quadranten ein äußerer Schaden vor. Wenn die Faulstelle überwiegend in den inneren 60% des jeweiligen Quadranten liegt, liegt für diesen Quadranten ein innerer Schaden vor.

Daraus kann der Schwächungsfaktor bzw. die Schwächung des Biegemomentes ermittelt werden (siehe auch Tabelle 2).

Die Ermittlung des Schwächungsfaktors k für einen äußeren Schaden ergibt sich wie folgt:

Wenn nur bei einem Quadranten ein äußerer Schaden vorliegt, ist dies mit Schadensbild B gemäß Fig. 1 gleichzusetzen, wenn bei mindestens zwei Quadranten ein äußerer Schaden vorliegt, entspricht dies Schadensbild A in Fig. 1. Bei Ermittlung des Schwächungsfaktors k für jeweils gegenüberliegende Quadranten (Quadrant 1+3 und Quadrant 2+4) wird jeweils der schlechtere Schwächungsfaktor gezählt.

Die Ermittlung des Schwächungsfaktors k für einen inneren Schaden erfolgt entsprechend:

Wenn nur bei einem Quadranten ein innerer Schaden vorliegt, ist dies mit Schadensbild E gemäß Fig. 1 gleichzusetzen. Wenn bei zwei einander nicht gegenüberliegenden Quadranten ein innerer Schaden vorliegt, liegt Schadensbild D gemäß Fig. 1 vor. Wenn bei zwei einander gegenüberliegenden Quadranten ein innerer Schaden vorliegt, trifft Schadensbild C gemäß Fig. 1 zu. Danach erfolgt wiederum die Ermittlung des Schwächungsfaktors k.

Zu beachten ist, dass ein Mast sowohl einen äußeren als auch einen inneren Schaden aufweisen kann. Wenn sowohl ein äußerer als auch ein innerer Schaden vorliegt, wird ein Gesamtschwächungsfaktor als Produkt der beiden Schwächungsfaktoren zu $k_{ges} = k_A * k_I$ berechnet.

Wenn der Schwächungsfaktor k< 0,9 und k> 0,7 bzw. bei überwiegend äußerem Schaden bei k> 0,5 liegt und $k_A < k_I$, ist zwar Schadensklasse I unterschritten, Schadensklasse IV aber noch nicht erreicht, so dass eine Überprüfung auf Schadensklasse II anhand bekannter "Beurteilung von geschwächten Masten" mit folgenden bekannten Schritten erfolgen muss:

a. Berechnung des zulässigen Biegemomentes: $\mathbf{M}$ = Mo * $k_{ges}$, wobei Mo = 1,45*D³*n/32
b. Ermittlung der Windkraft $\mathbf{W}$ abhängig von Durchmesser und Masthöhe
c. Berechnung des Nutzzuges: $\mathbf{N}$ = M/h - W (Biegemoment / Masthöhe - Windkraft)
d. Ermittlung der Belastung des Mastes an der Spitze $\mathbf{P}$ abhängig von Leiterseilquerschnitt und Spannweite.

Damit der Mast der Standsicherheit der Schadensklasse II entspricht, müssen folgende zwei Bedingungen erfüllt sein:

• $\mathbf{N >= P}$ - Der Nutzzug muss mindestens gleich der äußeren Belastung auf den Mast sein.

• $\mathbf{M >= 123*h}$ - Mindestwert für das Biegemoment.

Die allgemeine Ermittlung der Schadensklasse erfolgt gemäß untenstehender Tabelle, wie bereits weiter oben erläutert:

| Schadensklasse | Schwächung Biegemoment | Bedingungen erfüllt |
|---|---|---|
| I | weniger als 10% (k>=0,9) | Ja |
| II | 10-30% bei inneren Schäden (k= 0,9 bis 0,7) | Ja |
| | 10-50% bei äußeren Schäden (k= 0,9 bis 0,5) | |
| III | 10-30% bei inneren Schäden (k= 0,9 bis 0,7) | Nein |
| | 10-50% bei äußeren Schäden (k= 0,9 bis 0,5) | |
| IV | Mehr als 30% bei inneren Schäden (k<0,7) | Nein |
| | Mehr als 50% bei äußeren Schäden (k<0,5) | |

Somit erfolgt einerseits eine Beurteilung der Standsicherheit von Holzmasten bei Fäulnis nach den VDE-Vorschriften und andererseits anhand des berechneten σ -Wertes. Die schlechtere der beiden Bewertungen wird abschließend verwendet.

**Patentansprüche**

**1.** Verfahren zur Untersuchung und Beurteilung der Standfestigkeit von Holzmasten, insbesondere von Telefon- oder Strom masten, umfassend die folgenden Merkmale:

- Ermittlung von Messkennlinien durch Bohrwiderstandsmessungen am Holzmast, die dem tatsächlichen Bohrwiderstand oder einer daraus abgeleiteten Größe für die Holzfestigkeit oder die Holzdichte entsprechen,
- Ermittlung von Referenzkennlinien des Bohrwiderstands oder einer daraus abgeleiteten Größe der unteren Grenze für die Belastbarkeit, und
- Ermittlung von Schadstellen, Schadensbildern und Schadensklassen aus dem Vergleich der Messkennlinien der Bohrwiderstandsmessungen und der Referenzkennlinien, wobei
- die Bohrwiderstandsmessungen an den Holzmasten in zumindest zwei im Winkel zueinander eingebrachten Bohrungen durchgeführt werden,

**dadurch gekennzeichnet, dass** zumindest eine Bohrung in einer Leitungsrichtung und zumindest eine Bohrung 90° zur Leitungsrichtung darchgeführt werden.

**2.** Verfahren nach Anspruch 1, **dadurch gekennzeichnet, dass** das Verfahren die folgenden weiteren Merkmale umfasst:

- Sichtkontrolle der Holzmasten,
- Kombination der Ergebnisse der Bohrwiderstandsmessungen mit den Ergebnissen der Sichtkontrollen, und

- Festlegung der Intervalle für weitere Maßnahmen.

3. Verfahren nach Anspruch 1 oder 2, **dadurch gekennzeichnet, dass** Referenzkennlinien für verschiedene Holzarten aufgenommen werden.

4. Verfahren nach Anspruch 3, **dadurch gekennzeichnet, dass** für jede Holzart mindestens fünfzig Messkennlinien aufgenommen werden und daraus eine Referenzkennlinie ermittelt wird.

5. Verfahren nach einem der Ansprüche 1 bis 4, **dadurch gekennzeichnet, dass** zur Bestimmung eines unteren noch zulässigen Grenzwerts der Bruchfestigkeit eines Holzmasts eine 5%-Fraktile der Gaußschen Normalverteilung für die Referenzkennlinien herangezogen wird.

6. Verfahren nach einem der Ansprüche 1 bis 5, **dadurch gekennzeichnet, dass** der Vergleich einer Referenzkennlinie mit einer Messkennlinie abschnittsweise erfolgt.

7. Verfahren nach Anspruch 6, **dadurch gekennzeichnet, dass** eine Berechnung und ein Vergleich der Flächen unter den Kennlinien in vorgegebenen Schritten, vorzugsweise in 2,5%-Schritten, erfolgt.

8. Verfahren nach Anspruch 6 oder 7, **dadurch gekennzeichnet, dass** aus dem Vergleich einer Referenzkennlinie mit einer Messkennlinie eine Zuordnung zu einem Schadensbild und in eine Schadensklasse erfolgt.

9. Verfahren nach einem der Ansprüche 1 bis 8, **dadurch gekennzeichnet, dass** eine Kalibrierung der Bohrwiderstandswerte durch Ermittlung eines Skalierungswertes aus Mastumbruchsmessungen erfolgt.

10. Verfahren nach Anspruch 9, **dadurch gekennzeichnet, dass** der Skalierungswert der Steigung einer Geraden durch eine Wolke von Messpunkten in einem Diagramm entspricht, in welchem gemessene Biegespannungswerte gegen berechnete Biegespannungswerte aufgetragen sind.

11. Verfahren nach Anspruch 9 oder 10, **dadurch gekennzeichnet, dass** der Skalierungswert zwischen 2,0 und 2,5 beträgt.

12. Verfahren nach Anspruch 1, **dadurch gekennzeichnet, dass** an einem eingegrabenen Holzmast zumindest zwei 45°-Bohrungen im Erdübergangsbereich, davon eine Bohrung in einer Leitungsrichtung und eine Bohrung 90° zur Leitungsrichtung, durchgeführt werden.

13. Verfahren nach Anspruch 1, **dadurch gekennzeichnet, dass** an einem Holzmast mit Betonfuß zumindest zwei waagerechte Messungen zwischen den Befestigungen des Holzmasts an dem Betonfuß, davon eine Bohrung in einer Leitungsrichtung und eine Bohrung 90° zur Leitungsrichtung, durchgeführt werden.

14. Verfahren nach einem der Ansprüche 1 bis 13, **dadurch gekennzeichnet, dass** die Bohrwiderstandsmessung mittels eines Bohrers mit angeschliffener Spitze und einem Durchmesser von 1 bis 3 mm ausgeführt wird.

## Claims

1. A method for the examination and assessment of the stability of wooden poles, in particular of telephone poles or power poles, comprising the following features:

   - determining measurement characteristic curves by means of drill resistance measurements at the wooden pole, corresponding to the actual drill resistance or a variable derived therefrom for the wood stability or the wood density,
   - determining reference characteristic curves of the drill resistance or a variable derived therefrom of the lower limit for the strength thereof, and
   - determining faults, fault images or fault classes on the basis of the comparison of the measurement characteristic curves of the drill resistance measurements and the reference characteristic curves, wherein
   - the drill resistance measurements are performed at the wooden poles in at least two drillings introduced at an angle to each other,

**characterized in that** there is performed at least one drilling in a direction of the line and at least one drilling at 90° to the direction of the line.

2. A method according to claim 1, **characterized in that** the method comprises the following further features:

   - visual inspection of the wooden poles,
   - combination of the results of the drill resistance measurements with the results of the visual inspections, and
   - determination of the intervals for further measures.

3. A method according to claim 1 or 2, **characterized in that** reference characteristic curves are taken for different wood types.

4. A method according to claim 3, **characterized in that** there are taken at least fifty measurement characteristic curves for each wood type and that there is then determined a reference characteristic curve on the basis thereof.

5. A method according to any of claims 1 to 4, **characterized in that** a 5% fractile of the Gaussian distribution for the reference characteristic curves is used for determining the lower still permissible limit of the breaking resistance of a wooden pole.

6. A method according to any of claims 1 to 5, **characterized in that** the comparison of a reference characteristic curve with a measurement characteristic curve is realized portion per portion.

7. A method according to claim 6, **characterized in that** the areas under the characteristic curves are calculated and compared in predetermined increments, preferably in 2.5% increments.

8. A method according to claim 6 or 7, **characterized in that** there is performed an allocation to a fault image or into a fault class on the basis of the comparison of a reference characteristic curve with a measurement characteristic curve.

9. A method according to any of claims 1 to 8, **characterized in that** the drill resistance values are calibrated by determining a scaling value on the basis of the breaking pole measurements.

10. A method according to claim 9, **characterized in that** the scaling value corresponds to the gradient of a straight line through a cloud of measurement points in a diagram, wherein the measured bending stress values are applied versus calculated bending stress values.

11. A method according to claim 9 or 10, **characterized in that** the scaling value is between 2.0 and 2.5.

12. A method according to claim 1, **characterized in that** at least two 45° drillings in the ground transition area are performed at an embedded wooden pole, thereof one drilling in a direction of the line and one drilling at 90° to the direction of the line.

13. A method according to claim 1, **characterized in that** at least two horizontal measurements between the attachments of the wooden mast at the concrete socket are performed at a wooden pole with concrete socket, thereof one drilling in a direction of the line and one drilling at 90° in the direction of the line.

14. A method according to any of claims 1 to 13, **characterized in that** the drill resistance measurement is carried out using a driller having a bevelled tip and a diameter of 1 to 3 mm.

**Revendications**

1. Procédé destiné à la recherche et à l'évaluation de la solidité de mâts en bois, en particulier de mâts pour le téléphone et le courant électrique, comprenant les caractéristiques suivantes :

   - réalisation des courbes caractéristiques de mesure par des mesures de résistance au perçage sur le mât en bois, qui correspondent effectivement à la résistance au perçage, ou à une grandeur qui en est dérivée, pour évaluer la résistance du bois ou la densité du bois,

- réalisation de courbes caractéristiques de référence de la résistance au perçage, ou d'une grandeur qui en est dérivée, pour la limite inférieure de charge admissible, et

- recherche de zones de défauts, de types de défauts et de classes de défauts à partir de la comparaison des courbes caractéristiques de mesure des mesures de résistance au perçage et des courbes caractéristiques de référence,

- les mesures de résistance au perçage étant réalisées sur les mâts en bois par au moins deux perçages effectués à angle droit l'un par rapport à l'autre,

**caractérisé en ce**
qu'au moins un perçage est effectué dans une direction de la ligne et qu'au moins un perçage est effectué à 90 ° par rapport à la direction de la ligne.

2. Procédé selon la revendication 1 **caractérisé en ce que** le procédé comprend les autres caractéristiques suivantes :

- contrôle visuel des mâts en bois,
- comparaison des résultats des mesures de résistance au perçage avec les résultats des contrôles visuels, et
- établissement des échéances pour d'autres mesures.

3. Procédé selon les revendications 1 ou 2 **caractérisé en ce que** les courbes caractéristiques de référence sont enregistrées pour divers types de bois.

4. Procédé selon la revendication 3 **caractérisé en ce que**, pour chaque type de bois, au moins cinquante courbes caractéristiques sont enregistrées et qu'une courbe caractéristique de référence en est déduite.

5. Procédé selon l'une des revendications de 1 à 4 **caractérisé en ce que** pour la détermination d'une valeur limite inférieure admissible de la résistance à rupture d'un mât en bois, on considère les 5 % de fractale de la distribution normale gaussienne pour les courbes caractéristiques de référence.

6. Procédé selon l'une des revendications de 1 à 5 **caractérisé en ce que** la comparaison d'une courbe caractéristique de référence avec une courbe caractéristique de mesure est effectuée par tranches.

7. Procédé selon la revendication 6 **caractérisé en ce qu'**un calcul et qu'une comparaison des surfaces en dessous des courbes caractéristiques dans les étapes précédentes ont lieu, de préférence avec des pas de 2,5 %.

8. Procédé selon les revendications 6 ou 7 **caractérisé en ce qu'**à partir de la comparaison d'une courbe caractéristique de référence avec une courbe caractéristique de mesure, un classement a lieu par type de défaut et par classe de défaut.

9. Procédé selon l'une des revendications de 1 à 8 **caractérisé en ce qu'**un étalonnage des valeurs de résistance au perçage a lieu par la recherche d'une valeur d'échelle à partir des mesures de ruptures de mâts.

10. Procédé selon la revendication 9 **caractérisé en ce que** la valeur d'échelle de la pente d'une droite correspond à un nuage de points de mesure dans un diagramme dans lequel des valeurs de contraintes de flexion mesurées sont reportées en fonction de valeurs de contraintes de flexion calculées.

11. Procédé selon les revendications 9 ou 10 **caractérisé en ce que** la valeur d'échelle se situe entre 2,0 et 2,5.

12. Procédé selon la revendication 1 **caractérisé en ce qu'**au moins deux perçages à 45 ° dans la zone de transition avec la terre sont effectués, parmi ceux-ci, un perçage dans une direction de la ligne et un perçage à 90 ° par rapport à la direction de la ligne.

13. Procédé selon la revendication 1 **caractérisé en ce qu'**au moins deux mesures horizontales sont effectuées entre les fixations du mât en bois sur le pied en béton, dans le cas d'un mât en bois comprenant un pied en béton, parmi ces mesures, un perçage est effectué dans une direction de la ligne et un perçage est effectué à 90 ° par rapport à la direction de la ligne.

14. Procédé selon l'une des revendications de 1 à 13 **caractérisé en ce que** la mesure de la résistance au perçage est effectuée au moyen d'une perceuse avec une mèche effilée et d'un diamètre de 1 à 3 mm.

Fig. 1

Fig. 2A

System-Nr. 4 44 07 - Mast-Nr. 77 - Messung 1 - 45° Bohrung

Fig. 2B

Fig. 3A

| | RESI · Messungen | | Umbruchsversuch | |
|---|---|---|---|---|
| | Feld | Umbruch | | |
| Sigma ber. [MPa] | | | Sigma [MPa] | 17,40 |
| Bruchkraft ber. [N] | | | Bruchkraft [N] | 3902 |
| Sicherheit | | | Sicherheit | 1,81 |
| Schadensbild | | | Bruchstelle u. EOK [mm] | 0 |
| | | | Einspannung [mm] | -300 |
| Besonderheit | F | F | Asto bei Bruchstelle | Nein |
| Bewertung | ⊙ | ⊙ | Bewertung | ⊙ |
| F = Fäulnis, GD = Geringe Holzdichte | | | Nutzzug lt. ÖNORM [II] | 2152 |

Fig. 3B

Fig. 4A

Fig. 4B

Fig. 5

**System-Nr. 4 44 07  -  Mast-Nr.  -  Messung 1  -  90° Bohrung**

## Fig. 6A

**System-Nr. 4 44 07  -  Mast-Nr.  -  Messung 2  -  90° Bohrung**

## Fig. 6B

## Fig. 6C

14

**IN DER BESCHREIBUNG AUFGEFÜHRTE DOKUMENTE**

**In der Beschreibung aufgeführte Patentdokumente**

- WO 0165253 A2
- FR 2760842 A
- DE 4122494 A1